# EUROPEAN PATENT SPECIFICATION

(11) **EP 4 158 052 B1**
(45) Date of publication and mention of the grant of the patent: **30.09.2026**
(21) Application number: 21812454.3
(22) Date of filing: 19.05.2021
(51) Int. Cl.: C12Q 1/68, C12Q 1/6804, C12Q 1/6813, C12Q 1/34, C12Q 1/25, G01N 33/543

(54) **DETECTING ANALYTES IN A SAMPLE**
NACHWEIS VON ANALYTEN IN EINER PROBE
DÉTECTION D'UN ANALYTE DANS UN ÉCHANTILLON

(30) Priority: 29.05.2020 IN 202011022585
(43) Date of publication of application: 05.04.2023
(60) Divisional application: 26195169.3
(73) Proprietor: Siemens Healthcare Diagnostics, Inc., Tarrytown, NY 10591 (US)
(72) Inventor: KHANDIGE, Divya, Bangalore, Karnataka 560032 (IN); MITRA, Nivedita, Bangalore, Karnataka 560066 (IN); V M, Ramya, Bangalore, Karnataka 560019 (IN)
(74) Representative: Schweitzer, Klaus
(86) International application number: PCT/US2021/070578
(87) International publication number: WO 2021/243349

(56) References cited:
- WO-A1-03/102590
- WO-A1-2007/054515
- WO-A1-2012/039529
- WO-A2-2013/113699
- RU-C2- 2 423 136
- US-A1- 2011 244 457
- US-A1- 2013 196 316
- US-A1- 2014 274 775
- US-A1- 2019 383 798
- LANDEGREN ULF ET AL: "A myopic perspective on the future of protein diagnostics", NEW BIOTECHNOLOGY, ELSEVIER BV, NL, vol. 45, 5 January 2018 (2018-01-05), pages 14 - 18, XP085446769, ISSN: 1871-6784, DOI: 10.1016/J.NBT.2018.01.002
- RACHEL YUAN NONG ET AL: "Solid-phase proximity ligation assays for individual or parallel protein analyses with readout via real-time PCR or sequencing", NATURE PROTOCOLS, vol. 8, no. 6, 30 May 2013 (2013-05-30), pages 1234 - 1248, XP055184894, ISSN: 1754-2189, DOI: 10.1038/nprot.2013.070
- SPYROS DARMANIS ET AL: "Sensitive Plasma Protein Analysis by Microparticle-based Proximity Ligation Assays* □ S", 26 March 2009 (2009-03-26), pages 900248 - 200, XP055185083, Retrieved from the Internet <URL:http://www.mcponline.org/content/9/2/327.full.pdf> [retrieved on 20150422], DOI: 10.1074/mcp.M900248-MCP200
- HAMMOND MARIA ET AL: "Sensitive detection of aggregated prion protein via proximity ligation", vol. 8, no. 3, 4 May 2014 (2014-05-04), pages 261 - 265, XP055894606, ISSN: 1933-6896, Retrieved from the Internet <URL:https://www.ncbi.nlm.nih.gov/pmc/articles/PMC4601298/pdf/kprn-08-03-968465.pdf> DOI: 10.4161/pri.32231
- OLIVEIRA FELIPE MARQUES SOUZA DE ET AL: "Detection of post-translational modifications using solid-phase proximity ligation assay", NEW BIOTECHNOLOGY, vol. 45, 2018, pages 51 - 59, XP085446764, ISSN: 1871-6784, DOI: 10.1016/J.NBT.2017.10.005

## Description

### FIELD OF TECHNOLOGY

The present invention relates to a method of detecting analytes in a sample. In particular, the invention relates to quantifying the amount of analytes that may be present in a given sample.

### BACKGROUND

Effective and quick detection of clinically relevant analytes is the need of the hour. Early diagnosis of diseases requires the diagnostic methods to be sensitive to minute quantities of analytes in physiological samples such as whole blood, urine, sputum, etc. Current methods provide for early diagnosis of diseases. However, such methods involve multiple steps in the workflow, thereby increasing the complexity of the solution. Currently available methods such as antibody detection by agglutination-PCR (ADAP) involve binding of antibodies to synthetic antigen-DNA conjugates, enabling ligation of the DNA strands and subsequent quantification by quantitative PCR (qPCR). However, the specificity of this method may be low.

Rachel Yuan Nong ET AL: "Solid-phase proximity ligation assays for individual or parallel protein analyses with readout via real-time PCR or sequencing", (vol. 45, 5.01.2018) discloses solid-phase proximity ligation assays for protein detection. The proteins are captured via antibodies on a solid support by pairs of antibodies with attached DNA strands. Upon recognition by these sets of three antibodies, pairs of DNA strands are brought in proximity and are joined by ligation. Two oligonucleotides which are attached to antibodies that have bound the target are joined by ligation by adding a connector oligonucleotide and a ligase (in the case of PLA). All antibodies are first immobilized on the solid support in order to act as capture agents for the protein.
Spyros Darmanis ET AL: "Sensitive Plasma Protein Analysis by Microparticle-based Proximity Ligation Assays", (2009-03-26)) describes solid-phase proximity ligation assays using a plurality of antibodies immobilized on a solid support - for example directly on the walls of PCR tubes. The solid-phase proximity ligation assay is suitable for validation of a variety of protein biomarkers over broad dynamic ranges in clinical samples.

Therefore, there exists a need for a method that enables detection of analytes in a sample in minute quantities, that is effective and simple.

The object of the invention is therefore to provide a method that enables detection of analytes such as proteins in a given sample that is quick, effective and has a high sensitivity.

### SUMMARY

The invention is set out in the appended set of claims.

The invention achieves the object by a method of detection of one or more analytes in a sample. The method includes introducing the sample to a surface bound to one or more first antibodies, wherein the surface is bound to at least one portion of each first antibody. The first antibody is chosen such that the affinity of the first antibody towards the one or more analyte in the sample is high. The first antibody binds to the analyte in the sample to form a first antibody-analyte complex. The method further includes incubating the first antibody-analyte complex with at least one portion of a second antibody. The second antibody is conjugated with a nucleic acid fragment comprising an exposed 3' hydroxyl group, while another second antibody is conjugated with a nucleic acid fragment comprising an exposed 5' phosphate group. In an embodiment, multiple second antibodies bind with the first antibody-analyte complex to form first antibody-analyte-second antibody complex. The method enables the first antibody-analyte-second antibody complex which in turn helps in bringing the second antibody tethered nucleic acids in close proximity.

The method further includes ligating the nucleic acid fragment comprising the exposed 3' hydroxyl group with the nucleic acid fragment comprising the exposed 5' phosphate group. In an embodiment, the nucleic acid fragment comprising the 3' hydroxyl group may be in proximity to the nucleic acid fragment comprising the 5' phosphate group. The method further includes separating the ligated nucleic acid fragments from the first antibody-analyte-second antibody complex. The separation may be achieved, for example, via alkaline hydrolysis. Alternatively, the ligated nucleic acid fragments may be separated during an amplification process of the nucleic acid fragments. The separation is achieved such that the nucleic acid fragments conjugated to the second antibody is dissociated from the second antibody. The method further includes amplifying the separated ligated nucleic acid fragments and detecting the one or more analytes present in sample based on the amplified nucleic acid fragments. The nucleic acid hence acts as a surrogate for the target analyte.

The present invention is advantageous over ADAP in that the present invention uses at least one portion of the first antibody bound to the surface. This avoids binding of the antibody to more than one antigen. Additionally, the present invention includes a second antibody which binds to the first antibody-analyte complex. This improves the sensitivity with which the analytes are detected in the sample.

According to an embodiment, the nucleic acid fragment comprising the exposed 3' hydroxyl group and the nucleic acid fragment comprising the 5' exposed phosphate group are in close proximity to each other. Therefore, ligation of the two nucleic acid fragments is enabled due to proximity.

According to another embodiment, ligating the nucleic acid fragments includes introducing a linker nucleic acid fragment to the first antibody-analyte-second antibody complex. The linker nucleic acid fragment may be complementary to the nucleic acid fragment comprising the exposed 3' hydroxyl group and the nucleic acid fragment comprising the 5' exposed phosphate group. A ligase enzyme may be added to the linker nucleic acid and the first antibody-analyte-second antibody complex to form a mixture. The ligase enzyme catalyzes the ligation of the two nucleic acid fragments and the linker nucleic acid fragment. The mixture is further incubated so as to enable the linker nucleic acid fragment to connect the nucleic acid fragment comprising the exposed 3' hydroxyl group and the nucleic acid fragment comprising the 5' exposed phosphate group.

According to yet another embodiment, amplifying the ligated nucleic acid fragments includes performing an amplification that is quantitative and includes polymerase chain reaction or isothermal amplification on the ligated nucleic acid fragments.

According to a further embodiment, the method further includes pre-amplifying the ligated nucleic acid fragments before performing a quantitative amplification. Pre-amplification of the ligated nucleic acid fragments enables an increase in the sensitivity of the quantitative polymerase chain reaction or any other amplification method.

According to another embodiment, the surface to which the first antibody is bound is a spherical bead. Therefore, the spherical bead may be uniformly coated with the antibodies.

According to yet another embodiment, the sample may be chosen from a group including whole blood, sputum, urine, cerebrospinal fluid and bronchoalveolar lavage.

In another aspect, the invention relates to a kit for detecting one or more analytes in a sample. The kit includes a surface bound to one or more first antibodies, wherein the surface is bound to at least one portion of each first antibody, a second antibody conjugated to a nucleic acid fragment comprising an exposed 3' hydroxyl group, and another second antibody conjugated to a nucleic acid fragment comprising an exposed 5' phosphate group. The kit comprises an alkaline agent for hydrolyzing at least one oligonucleotide which binds the nucleic acid fragment comprising the exposed 3' hydroxyl group and the nucleic acid fragment comprising the 5' exposed phosphate group to the at least one portion of the second antibody thereby separating the ligated nucleic acid fragments.

According to an embodiment, the kit further includes one or more enzymes for ligating the nucleic acid fragments conjugated to the secondary antibody.

According to an embodiment, the kit may further include one or more linker nucleic acid fragments that may be complementary to the nucleic acid fragment comprising the exposed 3' hydroxyl group and the nucleic acid fragment comprising the exposed 5' phosphate group.

### BRIEF DESCRIPTION OF THE DRAWINGS

The present invention is further described hereinafter with reference to illustrated embodiments shown in the accompanying drawings, in which:
Figure 1 illustrates a flowchart of a method of detecting one or more analytes in a sample, according to an embodiment of the invention.
Figure 2A illustrates a schematic diagram of a method of detecting one or more analytes in a sample, according to an embodiment of the invention.
Figure 2B illustrates a schematic diagram of a method of detecting one or more analytes in a sample, according to another embodiment of the invention.
Figure 3 illustrates a flowchart of a method of ligating the nucleic acid fragments, according to an embodiment.

### DETAILED DESCRIPTION

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

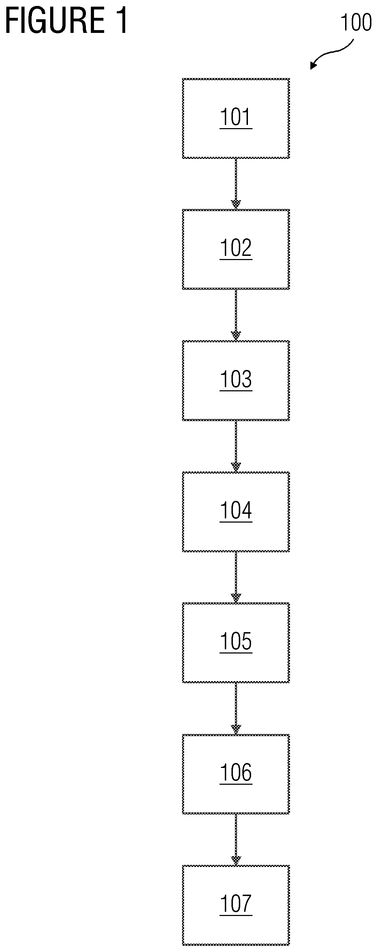

Figure 1 illustrates a flowchart of a method 100 of detecting one or more analytes in a given sample. The analytes, may be, for example, any antigen for which one or more antibodies can be produced. The analytes may include, for example, pathogens such as bacteria, viruses, etc., protein molecules associated with pathogens, autoantigens generated by an individual as a result of an autoimmune disorder, protein biomarkers for any specific disease present in physiological fluids etc. The sample may be any physiological fluid such as whole blood, urine, sputum, bronchoalveolar lavage, cerebrospinal fluid, etc. that may be capable of carrying the one or more analytes. At step 101 of the method 100, the sample comprising the one or more analytes is introduced to a surface bound to at least one portion of a first antibody. The surface may possess properties such that multiple bound first antibodies are in close proximity to each other. The surface may be, for example, spherical in shape thereby allowing the at least one portion of multiple first antibodies to be in close proximity to each other. The at least one portion of the first antibody binds with the one or more analytes present in the sample to form a first antibody-analyte complex. The first antibody is chosen such that the first antibody has an affinity to the one or more analytes present in the sample. The first antibody may also include, for example, aptamers, nanobodies, or any other derivatives of antibodies. In an embodiment, the surface may be bound to the one or more first antibody as a whole component. In another embodiment, the surface may be bound to the first antibody such that an antigen binding fragment (Fab) of the first antibody is exposed. At step 102 of the method 100, the first antibody-analyte complex is incubated with second antibodies. The concentration of the second antibodies may be stoichiometrically determined. Each second antibody may be conjugated with one or more nucleic acid fragment. The second antibody may be chosen such that the second antibody has an affinity for the one or more analytes present in the sample. The at least one portion of each second antibody binds to the first antibody-analyte complex to form an agglutination complex or a first antibody-analyte-second antibody complex such that the analyte is sandwiched between the first antibody and the second antibody. The one or more nucleic acid fragments conjugated to each second antibody may have at least one of an exposed 3' hydroxyl group or an exposed 5' phosphate group. In an embodiment, the properties of the surface to which the at least one portion of the first antibody is bound further enables increased proximity between the nucleic acid fragments conjugated to the at least one portion of the second antibody.

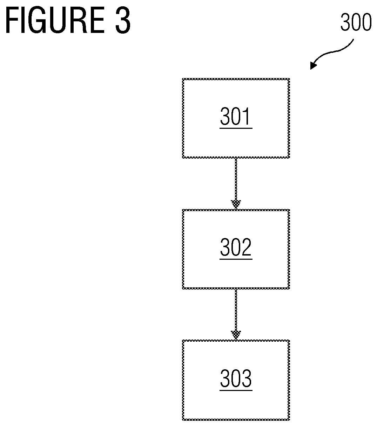

At step 103 of the method 100, the one or more nucleic acid fragments conjugated to the at least one portion of the second antibody are ligated. The method steps related to ligation of the one or more nucleic acid fragments is described in detail in Figure 3. Referring to Figure 3, at step 301 of method 300, one or more linker nucleic acid fragments is introduced to the first antibody-analyte-second antibody complex. The linker nucleic acid fragment may be a short nucleic acid fragment composed of 10-15 nucleotides. In an embodiment, the linker nucleic acid fragment may be chosen such that the linker nucleic acid fragment is complementary to the one or more nucleic acid fragments conjugated to the at least one portion of the second antibody. In a further embodiment, the linker nucleic acid fragment may be partly complementary to the nucleic acid fragment including the exposed 3' hydroxyl group and partly complementary to the nucleic acid fragment including the exposed 5' phosphate group. At step 302, ligase enzyme and the linker nucleic acid fragment is added to the first antibody-analyte-second antibody complex to form a mixture. Ligase enzyme acts as a catalyst in ligation of the nucleic acid fragments conjugated to the at least one portion of the second antibody to the linker nucleic acid fragment. At step 403, the mixture is incubated such that the linker nucleic acid fragment binds the nucleic acid fragment comprising the exposed 3' hydroxyl group and the nucleic acid fragment comprising the 5' exposed phosphate group. In an embodiment, the mixture may be incubated for about one minute at 50-60°C to enable hybridization of linker DNA to the oligos, also thereby bringing the exposed ends of the nucleic acid fragments proximal to each other. Advantageously, the properties associated with the surface to which the at least one portion of the first antibody is bound provides for effective ligation of the nucleic acid fragments conjugated to the second antibody due to the proximity of nucleic acid induced by the surface bound first antibody-analyte-second antibody complex. Due to the surface properties, the nucleic acid fragments bound to the second antibody are in close proximity to each other, thereby enabling effective ligation.

At step 104 of the method 100, the ligated nucleic acid fragments are separated from the first antibody-analyte-second antibody complex. In an embodiment, the one or more nucleic acid fragments may be conjugated to the second antibody through a bridging oligonucleotide. Therefore, the bridging oligonucleotide may be broken down to separate the ligated nucleic acid fragments from the first antibody-analyte-second antibody complex. Alternatively, the separation of the ligated nucleic acid fragments may also be achieved during the process of amplification. At step 105, the nucleic acid fragments are pre-amplified using polymerase chain reaction. The polymerase chain reaction based pre-amplification of the separated nucleic acid fragments enables increase in the sensitivity of the detection of the analytes in the sample. The process of pre-amplification of nucleic acid fragments using polymerase chain reaction is well known in the art and is therefore not elaborated upon in the description. In an alternate embodiment, the nucleic acid fragments may be amplified using any other amplification method. At step 106, the ligated nucleic acid fragments are amplified. This amplification may be performed using for example, real-time polymerase chain reaction. Real-time polymerase chain reaction or quantitative polymerase chain reaction enables real-time determination of quantity of the amplified nucleic acid. In an embodiment, real-time polymerase chain reaction may use a fluorescent dye labelled probe during the process of amplification of the nucleic acid fragments. As the number of copies of the amplified nucleic acid fragments increase, intensity of fluorescence generated also increases. At step 107, the one or more analytes present in the sample is detected. The detection may be based on, for example, the amount of fluorescence generated through real-time polymerase chain reaction.

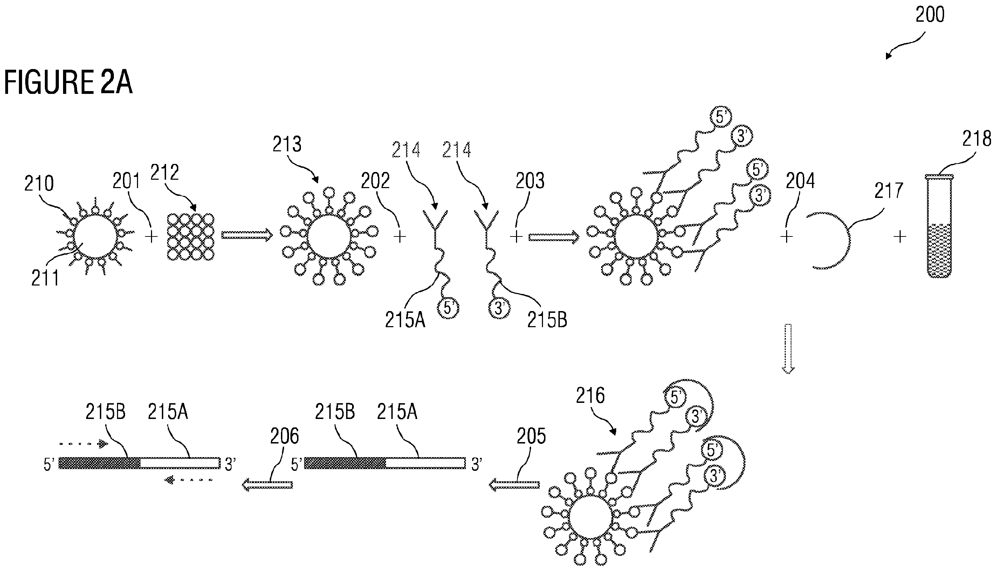

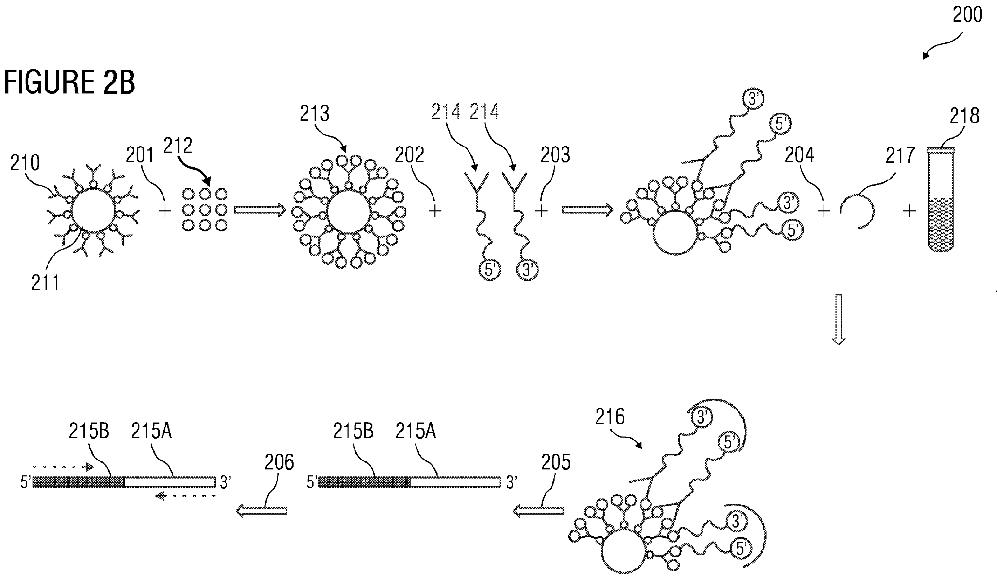

Figure 2A and 2B illustrate schematic diagrams of the method 200 of detecting one or more analytes in a sample, according to two embodiments. Figure 2A depicts a first antibody 210 as an analyte-specific Fab molecule conjugated to the surface 211 of a bead or dendrimer-like structure, while Figure 2B depicts a first antibody 210 as an analyte-specific antibody molecule conjugated to the surface 211 of a bead or dendrimer-like structure. One portion of a first antibody 210 is bound to a surface 211 such that another portion of the first antibody 210 is exposed to bind to one or more analytes 212 in the sample. In Figure 2A, the exposed portion of the first antibody 210 is an antigen binding fragment (Fab) region of the first antibody. The surface 211 may be spherical in shape and may be composed of polystyrene, silica, glass, etc. The spherical shape of the surface 211 enables effective agglutination of the at least one portion of the first antibody 210 with the one or more analytes 212 in the sample. The surface 211 may also include, for example, antibody dendrimers. The dendrimeric surface 211 has a spherical core and a dendritic structure or branches originating from the spherical core. Advantageously, the dendrimeric surface is capable of binding to a plurality of first antibodies. Therefore, antibody exposure to analytes present in the sample is increased. In an alternate embodiment, as illustrated in Figure 2B, the first antibody 210 may be bound to the surface 211 as a whole component. At step 201, the analytes 212 bind to the first antibody 210 when exposed to the surface 211 bound with at least one portion of the first antibody 210. A first antibody-analyte complex 213 is formed. At step 202, a set of second antibodies 214 is introduced to the first antibody-analyte complex 213, where the set of second antibodies is conjugated with two half DNA oligos: one half-oligo has a 5' phosphate exposed group (215A), while the second half has a 3' hydroxyl exposed group (215B). The second antibodies 214 are chosen such that each second antibody has an affinity for the one or more analytes 212. Each second antibody 214 is respectively conjugated to one or more nucleic acid fragments 215A, 215B. The nucleic acid fragments may include at least one of an exposed 3' hydroxyl group or an exposed 5' phosphate group. On incubation, the second antibodies 214 along with the one or nucleic acid fragments 215A, 215B bind to the first antibody-analyte complex 213 to generate first antibody-analyte-second antibody complex 216. At step 203, one or more linker nucleic acid fragments 217 and ligase enzyme 218 are added to the first antibody-analyte-second antibody complex 216. The linker nucleic acid fragments 217 is chosen such that each nucleic acid fragments 215A, 215B are complementary to the linker nucleic acid fragments 217, preferably 10-15 base pairs in length. The ligase enzyme 218 acts as a catalyst in the ligation process, with an incubation time of around 5-15 minutes from ligase enzyme addition, thereby resulting in ligating the nucleic acid fragments 215A, 215B conjugated to the second antibody 214 with the linker nucleic acid fragment 217 acting as a connecting agent. Advantageously, due to the spherical or dendrimeric format of the surface 211, the nucleic acid fragments 215A, 215B conjugated to the second antibodies 214 come in close proximity to each other in the first antibody-analyte-second antibody complex 216. Therefore, the ligation process is made more effective due to proximity. At step 204, the ligated nucleic acid fragments 215A, 215B are separated from the first antibody-analyte-second antibody complex 216, for example, through selective hydrolysis, and subjected to pre-amplification using polymerase chain reaction. The pre-amplification step improves the sensitivity of detection of analytes 212 present in the sample. At step 205, the pre-amplified nucleic acid fragments 215A, 215B are amplified using real-time polymerase chain reaction or any other isothermal amplification method. At step 206, the fluorescence generated during the real-time polymerase chain reaction is used to detect and quantify the one or more analytes 212 present in the sample.

The advantage of the invention is that sensitivity of detection of the one or more analytes in the sample in improved. Therefore, the invention enables detection of analytes in the sample to the level of picogram/µL and femtogram/µL. Additionally, the invention eliminates the need for multiple wash steps to remove background nucleic acid information. Therefore, the method steps can be carried out using a single equipment without intervening wash steps. Furthermore, the invention is compatible with molecular test platform. Therefore, nucleic acids and proteins may be detected using a single platform. This enables ease of workflow and allows for single sample collection for detection of nucleic acids and proteins.

The foregoing examples have been provided merely for the purpose of explanation. While the invention has been described with reference to various embodiments, it is understood that the words, which have been used herein, are words of description and illustration. The scope of the invention is defined by the appended claims.

## Claims

1. A method of detecting one or more analytes in a sample, the method comprising:
introducing the sample to a surface (211) bound to one or more first antibodies (210), wherein the surface (211) is bound to at least one portion of each first antibody to form a first antibody-analyte complex (213);
forming a first antibody-analyte-second antibody complex (216) by incubating the first antibody-analyte complex (213) with (a) at least one portion of a second antibody (214) conjugated with a nucleic acid fragment comprising an exposed 3' hydroxyl group (215B) and (b) at least one portion of another second antibody conjugated with a nucleic acid fragment comprising an exposed 5' phosphate group (215A);
ligating the nucleic acid fragment comprising the exposed 3' hydroxyl group (215B) and the nucleic acid fragment comprising the exposed 5' phosphate group (215A);
separating the ligated nucleic acid fragments (215A, 215B) from the first antibody-analyte-second antibody complex (216);
amplifying the separated ligated nucleic acid fragments (215A, 215B); and
detecting the one or more analytes present in the sample based on the amplified nucleic acid fragments, wherein the nucleic acid fragment comprising the exposed 3' hydroxyl group (215B) and the nucleic acid fragment comprising the exposed 5' phosphate group (215A) are in close proximity to each other.

2. The method according to claim 1, wherein the nucleic acid fragment comprising the exposed 3' hydroxyl group (215B) and the nucleic acid fragment comprising the exposed 5' phosphate group (215A) are ligated through enzymatic or chemical process.

3. The method according to claim 2, wherein ligating the nucleic acid fragment comprising the exposed 3' hydroxyl group (215B) and the nucleic acid fragment comprising the 5' exposed phosphate group (215A) through enzymatic process further comprises:
introducing one or more linker nucleic acid fragment (217) to the first antibody-analyte-second antibody complex (216);
adding ligase enzyme (218) to the linker nucleic acid fragment (217) and the first antibody-analyte-second antibody complex (216) to form a mixture; and
incubating the mixture to ligate the nucleic acid fragment comprising the exposed 3' hydroxyl group (215B) and the nucleic acid fragment comprising the 5' exposed phosphate group (215A), wherein the linker nucleic acid fragment (217) connects the nucleic acid fragment comprising the exposed 3' hydroxyl group (215B) and the nucleic acid fragment comprising the 5' exposed phosphate group (215A).

4. The method according to claim 1, wherein amplifying the ligated nucleic acid fragments (215A, 215B) comprises performing a quantitative amplification on the ligated nucleic acid fragments.

5. The method according to claim 4, further comprising pre-amplifying the ligated nucleic acid fragments (215A, 215B) before performing the quantitative amplification.

6. The method according to claim 1, wherein the surface (211) to which the one or more first antibodies are bound is at least one of spherical or dendrimer in format.

7. The method according to claim 1, wherein the sample is chosen from a group comprising but not limited to whole blood, sputum, urine, cerebrospinal fluid, and bronchoalveolar lavage.

8. A kit for detecting one or more analytes in a sample, the kit comprising:
a surface (211) bound to one or more first antibodies as specified in claim 1, wherein the surface is bound to at least one portion of each first antibody;
a second antibody (214) conjugated with a nucleic acid fragment comprising an exposed 3' hydroxyl group (215B) as specified in claim 1;
another second antibody (214) conjugated with a nucleic acid fragment comprising an exposed 5' phosphate group (215A) as specified in claim 1; and
an alkaline agent for hydrolyzing at least one oligonucleotide which binds the nucleic acid fragment comprising the exposed 3' hydroxyl group (215B) and the nucleic acid fragment comprising the 5' exposed phosphate group (215A) to the at least one portion of the second antibody (214) thereby separating the ligated nucleic acid fragments as specified in claim 1.

9. The kit according to claim 8, further comprising one or more enzymes for ligating the nucleic acid fragment comprising the exposed 3' hydroxyl group (215B) and the nucleic acid fragment comprising the exposed 5' phosphate group (215A).

10. The kit according to claim 8, further comprising one or more linker nucleic acid fragments (217) complementary to the nucleic acid fragment comprising the exposed 3' hydroxyl group (215B) and the nucleic acid fragment comprising the exposed 5' phosphate group (215A).

## Patentansprüche

1. Verfahren zum Erfassen eines oder mehrerer Analyten in einer Probe, wobei das Verfahren umfasst:
Einführen der Probe zu einer Oberfläche (211), die an einen oder mehrere erste Antikörper (210) gebunden ist, wobei die Oberfläche (211) an wenigstens einen Teil jedes ersten Antikörpers gebunden ist, um einen erster-Antikörper-Analyt-Komplex (213) zu bilden;
Bilden eines erster-Antikörper-Analyt-zweiter-Antikörper-Komplexes (216) durch Inkubieren des erster-Antikörper-Analyt-Komplexes (213) mit (a) wenigstens einem Teil eines zweiten Antikörpers (214), der mit einem Nukleinsäurefragment konjugiert ist, das eine freiliegende 3'-Hydroxygruppe (215B) umfasst, und (b) wenigstens einem Teil eines weiteren zweiten Antikörpers, der mit einem Nukleinsäurefragment konjugiert ist, das eine freiliegende 5'-Phosphatgruppe (215A) umfasst;
Ligieren des Nukleinsäurefragments, das die freiliegende 3'-Hydroxygruppe (215B) umfasst, und des Nukleinsäurefragments, das die freiliegende 5'-Phosphatgruppe (215A) umfasst;
Trennen der ligierten Nukleinsäurefragmente (215A, 215B) von dem erster-Antikörper-Analyt-zweiter-Antikörper-Komplex (216);
Amplifizieren der getrennten ligierten Nukleinsäurefragmente (215A, 215B); und
Erfassen des einen oder der mehreren Analyten, die in der Probe vorhanden sind, auf der Grundlage der amplifizierten Nukleinsäurefragmenten, wobei sich das Nukleinsäurefragment, das die freiliegende 3'-Hydroxygruppe (215B) umfasst, und das Nukleinsäurefragment, das die freiliegende 5'-Phosphatgruppe (215A) umfasst, in unmittelbarer Nähe zueinander befinden.

2. Verfahren nach Anspruch 1, wobei das Nukleinsäurefragment, das die freiliegende 3'-Hydroxygruppe (215B) umfasst, und das Nukleinsäurefragment, das die freiliegende 5'-Phosphatgruppe (215A) umfasst, durch enzymatisches oder chemisches Verfahren ligiert werden.

3. Verfahren nach Anspruch 2, wobei Ligieren des Nukleinsäurefragments, das die freiliegende 3'-Hydroxygruppe (215B) umfasst, und des Nukleinsäurefragments, das die 5'-freiliegende Phosphatgruppe (215A) umfasst, durch enzymatisches Verfahren ferner umfasst:
Einführen eines oder mehrerer Linker-Nukleinsäurefragmente (217) in den erster-Antikörper-Analyt-zweiter-Antikörper-Komplex (216);
Zugeben von Ligaseenzym (218) zu dem Linker-Nukleinsäurefragment (217) und dem erster-Antikörper-Analyt-zweiter-Antikörper-Komplex (216), um ein Gemisch zu bilden; und
Inkubieren des Gemischs, um das Nukleinsäurefragment, das die freiliegende 3'-Hydroxygruppe (215B) umfasst, und das Nukleinsäurefragment, das die 5'-freiliegende Phosphatgruppe (215A) umfasst, zu ligieren, wobei das Linker-Nukleinsäurefragment (217) das Nukleinsäurefragment, das die freiliegende 3'-Hydroxygruppe (215B) umfasst, und das Nukleinsäurefragment, das die 5'-freiliegende Phosphatgruppe (215A) umfasst, verbindet.

4. Verfahren nach Anspruch 1, wobei Amplifizieren der ligierten Nukleinsäurefragmente (215A, 215B) Durchführen einer quantitativen Amplifikation an den ligierten Nukleinsäurefragmenten umfasst.

5. Verfahren nach Anspruch 4, ferner umfassend Voramplifizieren der ligierten Nukleinsäurefragmente (215A, 215B) vor Durchführen der quantitativen Amplifikation.

6. Verfahren nach Anspruch 1, wobei die Oberfläche (211), an die der eine oder die mehreren ersten Antikörper gebunden sind, in dem Format von wenigstens einem von sphärisch und dendrimer vorliegt.

7. Verfahren nach Anspruch 1, wobei die Probe ausgewählt ist aus einer Gruppe umfassend, aber nicht darauf beschränkt, Vollblut, Sputum, Urin, Cerebrospinalfluid und bronchoalveoläre Lavage.

8. Kit zum Erfassen eines oder mehrerer Analyten in einer Probe, wobei das Kit umfasst:
eine Oberfläche (211), die an einen oder mehrere erste Antikörper nach Anspruch 1 gebunden ist, wobei die Oberfläche an wenigstens einen Teil jedes ersten Antikörpers gebunden ist;
einen zweiten Antikörper (214), konjugiert mit einem Nukleinsäurefragment, das eine freiliegende 3'-Hydroxygruppe (215B) umfasst, nach Anspruch 1;
einen weiteren zweiten Antikörper (214), konjugiert mit einem Nukleinsäurefragment, das eine freiliegende 5'-Phosphatgruppe (215A) umfasst, nach Anspruch 1; und
ein alkalisches Mittel zum Hydrolysieren wenigstens eines Oligonukleotids, das das Nukleinsäurefragment, das die freiliegende 3'-Hydroxygruppe (215B) umfasst, und das Nukleinsäurefragment, das die 5'-freiliegende Phosphatgruppe (215A) umfasst, an den wenigstens einen Teil des zweiten Antikörpers (214) bindet, um die ligierten Nukleinsäurefragmente nach Anspruch 1 zu trennen.

9. Kit nach Anspruch 8, ferner umfassend ein oder mehrere Enzyme zum Ligieren des Nukleinsäurefragments, das die freiliegende 3'-Hydroxygruppe (215B) umfasst, und des Nukleinsäurefragments, das die freiliegende 5'-Phosphatgruppe (215A) umfasst.

10. Kit nach Anspruch 8, ferner umfassend ein oder mehrere Linker-Nukleinsäurefragmente (217), die komplementär zu dem Nukleinsäurefragment, das die freiliegende 3'-Hydroxygruppe (215B) umfasst, und dem Nukleinsäurefragment, das die freiliegende 5'-Phosphatgruppe (215A) umfasst, sind.

## Revendications

1. Procédé de détection d'un ou de plusieurs analytes dans un échantillon, le procédé comprenant :
l'introduction de l'échantillon sur une surface (211) liée à un ou plusieurs premiers anticorps (210), la surface (211) étant liée à au moins une partie de chaque premier anticorps pour former un premier complexe anticorps-analyte (213) ;
la formation d'un premier complexe anticorps-analyte-second anticorps (216) en incubant le premier complexe anticorps-analyte (213) avec (a) au moins une partie d'un second anticorps (214) conjuguée à un fragment d'acide nucléique comprenant un groupe hydroxyle 3' exposé (215B) et (b) au moins une partie d'un autre second anticorps conjuguée à un fragment d'acide nucléique comprenant un groupe phosphate 5' exposé (215A) ;
la ligature du fragment d'acide nucléique comprenant le groupe hydroxyle 3' exposé (215B) et du fragment d'acide nucléique comprenant le groupe phosphate 5' exposé (215A) ;
la séparation des fragments d'acide nucléique ligaturés (215A, 215B) du premier complexe anticorps-analyte-second anticorps (216) ;
l'amplification des fragments d'acide nucléique ligaturés (215A, 215B) séparés ; et
la détection des un ou plusieurs analytes présents dans l'échantillon sur la base des fragments d'acide nucléique amplifiés, le fragment d'acide nucléique comprenant le groupe hydroxyle 3' exposé (215B) et le fragment d'acide nucléique comprenant le groupe phosphate 5' exposé (215A) étant à proximité l'un de l'autre.

2. Procédé selon la revendication 1, dans lequel le fragment d'acide nucléique comprenant le groupe hydroxyle 3' exposé (215B) et le fragment d'acide nucléique comprenant le groupe phosphate 5' exposé (215A) sont ligaturés par le biais d'un procédé enzymatique ou chimique.

3. Procédé selon la revendication 2, dans lequel la ligature du fragment d'acide nucléique comprenant le groupe hydroxyle 3' exposé (215B) et du fragment d'acide nucléique comprenant le groupe phosphate exposé 5' (215A) par le biais d'un processus enzymatique comprend en outre :
l'introduction d'un ou de plusieurs fragments d'acide nucléique de liaison (217) au premier complexe anticorps-analyte-second anticorps (216) ;
l'ajout d'une enzyme ligase (218) au fragment d'acide nucléique de liaison (217) et au premier complexe anticorps-analyte-second anticorps (216) pour former un mélange ; et
l'incubation du mélange pour ligaturer le fragment d'acide nucléique comprenant le groupe hydroxyle 3' exposé (215B) et le fragment d'acide nucléique comprenant le groupe phosphate 5' exposé (215A), le fragment d'acide nucléique de liaison (217) reliant le fragment d'acide nucléique comprenant le groupe hydroxyle 3' exposé (215B) et le fragment d'acide nucléique comprenant le groupe phosphate 5' exposé (215A).

4. Procédé selon la revendication 1, dans lequel l'amplification des fragments d'acide nucléique ligaturés (215A, 215B) comprend la réalisation d'une amplification quantitative sur les fragments d'acide nucléique ligaturés.

5. Procédé selon la revendication 4, comprenant en outre la pré-amplification des fragments d'acide nucléique ligaturés (215A, 215B) avant de réaliser l'amplification quantitative.

6. Procédé selon la revendication 1, dans lequel la surface (211) à laquelle les un ou plusieurs premiers anticorps sont liés est au moins un format parmi sphérique ou dendrimère.

7. Procédé selon la revendication 1, dans lequel l'échantillon est choisi dans un groupe comprenant, mais non limité à, du sang total, des expectorations, de l'urine, du liquide céphalo-rachidien, et un lavage broncho-alvéolaire.

8. Kit pour détecter un ou plusieurs analytes dans un échantillon, le kit comprenant :
une surface (211) liée à un ou plusieurs premiers anticorps tel que spécifié dans la revendication 1, la surface étant liée à au moins une partie de chaque premier anticorps ;
un second anticorps (214) conjugué à un fragment d'acide nucléique comprenant un groupe hydroxyle 3' exposé (215B) tel que spécifié dans la revendication 1 ;
un autre second anticorps (214) conjugué à un fragment d'acide nucléique comprenant un groupe phosphate 5' exposé (215A) tel que spécifié dans la revendication 1 ; et
un agent alcalin pour hydrolyser au moins un oligonucléotide qui lie le fragment d'acide nucléique comprenant le groupe hydroxyle 3' exposé (215B) et le fragment d'acide nucléique comprenant le groupe phosphate 5' exposé (215A) à l'au moins une partie du second anticorps (214) séparant ainsi les fragments d'acide nucléique ligaturés tel que spécifié dans la revendication 1.

9. Kit selon la revendication 8, comprenant en outre une ou plusieurs enzymes pour ligaturer le fragment d'acide nucléique comprenant le groupe hydroxyle 3' exposé (215B) et le fragment d'acide nucléique comprenant le groupe phosphate 5' exposé (215A).

10. Kit selon la revendication 8, comprenant en outre un ou plusieurs fragments d'acide nucléique de liaison (217) complémentaires du fragment d'acide nucléique comprenant le groupe hydroxyle 3' exposé (215B) et le fragment d'acide nucléique comprenant le groupe phosphate 5' exposé (215A).
